# EUROPÄISCHE PATENTSCHRIFT

(11) **EP 0 973 484 B1**
(45) Veröffentlichungstag und Bekanntmachung des Hinweises auf die Patenterteilung: **07.07.2004**
(21) Anmeldenummer: 97953710.7
(22) Anmeldetag: 28.11.1997
(51) Int. Cl.: A61K 7/00, A61K 7/48, A61K 9/107

(54) **ÖL-IN-WASSER-EMULSIONEN ZUR WIEDERHERSTELLUNG DER LAMELLARITÄT DER LIPIDSTRUKTUR GESCHÄDIGTER HAUT**
OIL-IN-WATER EMULSIONS FOR RECONSTITUTING LAMELLARITY OF THE LIPID STRUCTURE OF DAMAGED SKIN
EMULSIONS HUILE DANS L'EAU POUR LA RESTITUTION DE LA LAMELLARITE DE LA STRUCTURE LIPIDIQUE DE LA PEAU ENDOMMAGEE

(30) Priorität: 03.04.1997 DE 19713793
(43) Veröffentlichungstag der Anmeldung: 26.01.2000
(73) Patentinhaber: Henkel Kommanditgesellschaft auf Aktien, 40589 Düsseldorf-Holthausen (DE)
(72) Erfinder: BORDAT, Pascal, F-31130 Flourens (FR); ORTANDERL, Stefanie, D-41363 Jüchen (DE); KAMPMANN, Martina, D-41352 Korschenbroich (DE); WALDMANN-LAUE, Marianne, D-40789 Monheim (DE); KNÜBEL, Georg, D-40229 Düsseldorf (DE); MAUSBERG, Marcus, D-72160 Mühlen (DE)
(86) Internationale Anmeldenummer: PCT/EP1997/006639
(87) Internationale Veröffentlichungsnummer: WO 1998/044896

(56) Entgegenhaltungen:
- EP-A- 0 217 105
- EP-A- 0 312 343
- EP-A- 0 641 557
- WO-A-98/07406
- DE-A- 4 337 041

## Beschreibung

Die Erfindung betrifft sogenannte Lamellaremulsionen, deren Emulsionströpfchen von einer flüssig-kristallinen, lamellaren Phase aus Lipidmolekülen und Wasser umgeben und dadurch besonders stabilisiert und zur Wiederherstellung des gestörten Ordnungsgrades geschädigter Haut besonders geeignet sind.

Aus der Fachliteratur ist bekannt, daß solche Lamellaremulsionen den Wasserhaushalt der Haut günstig beeinflussen und eine hohe Feuchtigkeitsspeicherung in der Haut bewirken. Zur gezielten Herstellung von Lamellaremulsionen wird gemäß G. Dahms, Cosmetics & Toiletries, Vol. 101, Nov. 1986, Seiten 113 -115 angeregt, ein Öl mit einem Emulgator ähnlicher Struktur zu verwenden. In EP-A-0 641 557 wird empfohlen, als Emulgatorkomponenten ein lipophiles Tensid, ein hydrophiles Tensid und eine freie Fettsäure zu verwenden. In WO 94/17830 wird vorgeschlagen, als Emulgatoren Sorbitan- und Sucrose-Fettsäureester zu verwenden und gemäß WO 95/28913 A1 wird zusätzlich Harnstoff zur Herstellung solcher lamellarer Emulsionen eingesetzt.

Es wurde nunmehr festgestellt, daß für eine gezielte Herstellung von Öl-in-Wasser-Emulsionen mit anisotropen lamellaren Phasen weniger die Art des Öls oder des Emulgators als die Wahl des geeigneten Coemulgators ausschlaggebend ist.

Gegenstand der Erfindung ist daher eine Öl-in-Wasser-Emulsion mit lamellaren, flüssig-kristallinen Phasen, enthaltend eine kosmetische Öl- oder Fettkomponente, einen hydrophilen Emulgator und einen lipophilen Coemulgator, dadurch gekennzeichnet, daß als lipophiler Coemulgator ein Lipid der allgemeinen Formel R¹ - O - R² enthalten ist, in der R¹ eine primäre lineare Alkyl-, Alkenyl- oder Acylgruppe mit 20 - 30 C-Atomen und R² Wasserstoff eine Gruppe der Formel - (CₙH₂ₙO)ₓ - H, in der x = 1 oder 2 und n = 2 - 4 ist, oder eine Polyhydroxyalkylgruppe mit 4 - 6 C-Atomen und 2 - 5 Hydroxylgruppen ist.

Die erfindungsgemäßen Öl-in-Wasser-Emulsionen können als innere Phase entweder eine kosmetische Öl- oder Fettkomponente oder eine Wasser-in-Öl-Emulsion enthalten. Im letzteren Fall stellen die erfindungsgemäßen Lamellaremulsionen Wasser-in-Öl-in-Wasser-Emulsionen dar.

Der lipophile Coemulgator der Formel R¹ - O - R² ist bevorzugt ein Behen- oder Erucylderivat, in welchem R¹ eine lineare, endständig substituierte Alkyl-, Alkenyl- oder Acylgruppe mit 22 C-Atomen ist, in einer Menge von 10 - 90 Gew.-% der Ölphase. Besonders bevorzugt ist als lipophiler Coemulgator Behenylalkohol in einer Menge von 20 - 80 Gew.-% der gesamten Ölphase enthalten.

Weitere geeignete Coemulgatoren sind die Anlagerungsprodukte von 1 oder 2 Mol Ethylenoxid oder Propylenoxid an Behenylalkohol, Erucylalkohol, Arachidylalkohol oder auch an Behensäure oder Erucasäure. Schließlich eignen sich auch die Monoester von C₂₀-C₃₀-Fettsäuren mit Polyolen wie z.B. Pentaerythrit, Trimethylolpropan, Diglycerin, Sorbit, Glucose oder Methylglucose. Beispiele für solche Produkte sind z.B. Sorbitan-Monobehenat oder Pentaerythrit-monoerucat.

Als hydrophile Emulgatoren zur Herstellung der erfindungsgemäßen Öl-in-Wasser-Emulsionen eignen sich alle für die Emulgierung kosmetischer Öl- und Fettkomponenten geeigneten Tenside. Dies sind vor allem ionische Emulgatoren oder nichtionische Emulgatoren mit einem HLB-Wert von 8 bis 18. Der HLB-Wert stellt dabei einen Wert dar, der sich aus der Struktur des Moleküls nach der Formel HLB = 0,2 x (100 - L) ergibt, worin L der prozentuale Gewichtsanteil der lipophilen Alkyl-, Alkenyl- oder Acylgruppen im Molekül ist.

Als ionische Emulgatoren eignen sich anionische, kationische, zwitterionische und amphotere Tenside, bevorzugt solche mit einer primären, linearen Alkyl- oder Alkenylgruppe mit 12 - 18 C-Atomen. Geeignete anionische Emulgatoren sind z.B. die Salze von C₁₂C₁₈-Fettsäuren, von Schwefelsäuremonoestern oder Phosphorsäuremonoestern von C₁₂-C₁₈-Fettalkoholen, von C₁₂-C₁₈-Acylisethionsäuren, von C₁₂-C₁₈-Alkansulfonsäuren oder von C₁₂-C₁₈-Acylaminosäuren. Kationische Emulgatoren sind z.B. Cetyl-trimethylammonium-chlorid oder Distearoxyethyl-hydroxyethyl-methylammonium-chlorid. Geeignete zwitterionische Tenside sind z.B. Betaintenside wie Stearamidopropyl-dimethyl-carboxymethylammonium-betain und geeignete Amphotenside sind zB. Cetylaminopropionsäure oder Kokoamphocarboxyglycinat. Auch Aminoxidtenside eignen sich als hydrophile Emulgatoren.

Geeignete nichtionogene Tenside mit HLB-Werten von 8 bis 18 sind insbesondere die Anlagerungsprodukte von Ethylenoxid an Fettsäuren, an Fettalkohole, an Fettsäurealkanolamide, an Fettsäuremonoglyceride, an Sorbitanfettsäureester, an Methylglucosid-Fettsäureester oder an andere Lipide mit Carboxyl-, Hydroxyl- oder Aminogruppen, wobei der Anteil der dabei gebildeten Ethoxygruppen wenigstens 40 Gew.-% ausmachen sollte. Weitere geeignete nichtionische Tenside sind Alkylpolyglucoside, Zuckerester und Polyglycerin-Fettsäureester.

Als Öl- und Fettkomponenten eignen sich alle pflanzlichen, tierischen, mineralischen und synthetischen Öle, Fette und Wachse, die für eine Anwendung am menschlichen Körper aus physiologischen und ästhetischen Gründen infrage kommen. Geeignet sind z.B. Paraffine, Fettsäureester einwertiger oder mehrwertiger Alkohole, z.B. Triglyceride, Fettsäure-Fettalkohol-Ester, Fettsäure-Dicarbonsäure-Polyol-Polyester, Fettalkohol-Diol-Dicarbonsäure-Polyester, Di-n-alkylether, Polyolefine oder Silikonöle. Bevorzugt werden flüssige Öle oder Gemische aus Ölen und Wachsen eingesetzt, die bei 20°C flüssig sind. Als Ölkomponenten geeignete Monoester sind z.B. die Methylester und Isopropylester von Fettsäuren mit 12 - 22 C-Atomen, wie z.B. Methyllaurat, Methylstearat, Methyloleat, Methylerucat, Isopropylpalmitat, Isopropylmyristat, Isopropylpalmitat, Isopropylstearat, Isopropyloleat. Andere geeignete Monoester sind z.B. n-Butylstearat, n-Hexyllaurat, n-Decyloleat, Isooctylstearat, Isononylpalmitat, Isononyl-isononanoat, 2-Ethylhexylpalmitat, 2-Ethylhexyl-laurat, 2-Hexyldecyl-stearat, 2-Octyldodecyl-palmitat, Oleyloleat, Oleylerucat. Erucyloleat sowie Ester, die aus technischen aliphatischen Alkoholgemischen und technischen aliphatischen Carbonsäuren erhältlich sind, z.B. Ester aus gesättigten und ungesättigten Fettalkoholen mit 12 - 22 C-Atomen und gesättigten und ungesättigten Fettsäuren mit 12 - 22 C-Atomen, wie sie aus tierischen und pflanzlichen Fetten zugänglich sind. Geeignet sind auch natürlich vorkommende Monoester- bzw. Wachsester-Gemische, wie sie z.B. im Jojobaöl oder im Spermöl vorliegen.

Geeignete Dicarbonsäureester sind z.B. Di-n-butyl-adipat, Di-n-butyl-sebacat, Di-(2-ethylhexyl)-adipat, Di-(2-hexyldecyl)-succinat und Di-isotridecyl-acelaat. Geeignete Diolester (III) sind z.B. Ethylenglycol-dioleat, Ethylenglycol-di-isotridecanoat, Propylenglycol-di-(2-ethylhexanoat), Butandiol-di-isostearat und Neopentylglycol-di-caprylat.

Als Fettsäuretriglyceride können natürliche, pflanzliche Öle, z.B. Olivenöl, Sonnenblumenöl. Sojaöl, Erdnußöl, Rapsöl, Mandelöl, Palmöl, aber auch die flüssigen Anteile des Kokosöls oder des Palmkernöls sowie tierische Öle, wie z.B. Klauenöl, die flüssigen Anteile des Rindertalges-oder auch synthetische Triglyceride, wie sie durch Veresterung von Glycerin mit Fettsäuren mit 8 - 22 C-Atomen erhalten werden, z.B. Triglyceride von Caprylsäure-Caprinsäure-Gemischen, Triglyceride aus technischer Ölsäure oder aus Palmitinsäure-Gemischen eingesetzt werden.
Die Herstellung der erfindungsgemäßen Öl-in-Wasser-Emulsionen kosmetischer Öl- oder Fettkomponenten, die lamellare, flüssig-kristalline Phasen aufweisen, erfolgt in an sich bekannter Weise unter Verwendung hydrophiler Emulgatoren und lipophiler Coemulgatoren, wobei man die wäßrige Phase, die hydrophile Emulgatoren enthalten kann, mit der Öl- oder Fettphase, die als lipophile Coemulgatoren wenigstens ein Lipid der allgemeinen Formel R¹- O - R² enthält, in der R¹ eine lineare Alkyl-, Alkenyl- oder Acylgruppe mit 20 bis 30 C-Atomen und R² Wasserstoff, eine Gruppe der Formel - (CₙH₂ₙO)ₓ - H, in der x = 1 oder 2 und n = 2 - 4 ist, oder eine Polyhydroxyalkylgruppe mit 4 - 6 C-Atomen und 2 - 5 Hydroxylgruppen ist, intensiv vermischt.

Die wäßrige Phase enthält dabei alle wasserlöslichen Komponenten, z.B. einen wasserlöslichen Emulgator, die Konservierungsmittel, Puffersalze, Magnesiumchlorid, Propylenglycol, Glycerin, wasserlösliche polymere Verdickungsmittel oder wasserlösliche kosmetische Wirkstoffe.

Der Ölphase werden neben den kosmetischen Ölen und Fetten auch die öllöslichen Emulgatoren und insbesondere der lipophile Coemulgator zugegeben. Schließlich werden auch weitere, eventuell enthaltene öllösliche Hilfsstoffe, z.B. öllösliche Antioxidantien und Konservierungsstoffe, Wachse, Silikone und die öllöslichen kosmetischen Wirkstoffe der Ölphase zugesetzt. Die Ölphase wird dann auf eine Temperatur erhitzt, bei der sie als klare homogene Schmelze vorliegt. Auf die gleiche Temperatur wird auch die wäßrige Phase erwärmt. Sodann werden Ölphase und Wasserphase intensiv miteinander vermischt.

Wenn ein nichtionogener Emulgator verwendet wird, dessen Phaseninversionstemperatur unter 100°C liegt, so wird die Emulsion bevorzugt bei dieser Temperatur hergestellt oder beim Emulgieren auf diese Temperatur erwärmt. Dadurch wird die Emulsion zur Wasser-in-Öl-Emulsion, die dann beim Unterschreiten der Phaseninversionstemperatur wieder in eine O/W-Emulsion invertiert und dabei besonders feinteilig, niedrigviskos und lagerstabil anfällt.

Der Zusatz von Duftstoffen und besonders leichtflüchtigen oder temperaturempfindlichen Stoffen erfolgt bevorzugt erst nach dem Abkühlen auf Temperaturen von 40°C oder darunter.

Die erfindungsgemäßen Lamellaremulsionen können je nach Art und Menge der inneren Phase dünnflüssig bis cremeförmig sein. Die Konsistenz läßt sich auch durch Verdickungsmittel oder durch das Emulgierverfahren, also über die Feinteiligkeit, in gewissem Umfang steuern.

Die erfindungsgemäßen Öl-in-Wasser-Emulsionen behalten ihre Lamellarität unabhängig von dem Gewichtsverhältnis von Ölphase zu Wasserphase bei, die Öltröpfchen behalten also auch nach weitgehender Verdünnung mit Wasser ihre flüssig-kristalline Lipiddoppelschicht-Hülle. Sie läßt sich aufgrund ihrer doppelbrechenden Eigenschaften im polarisierten Licht sichtbar machen.

Die erfindungsgemäßen Lamellaremulsionen eignen sich zur Pflege der Haut. Sie erhöhen nicht nur das Feuchtigkeitsrückhaltevermögen der Haut, sondern erhöhen auch den Ordnungsgrad der Epidermis und verbessern die Barrierefunktion der Haut. Nach Hautschädigung, z.B. durch Tenside oder mechanische Beanspruchung, führt eine Behandlung mit der erfindungsgemäßen Lamellarcreme rascher zu einer Wiederherstellung der Lamellarität der epidermalen Lipidstrukturen.

Ein weiterer Gegenstand der Erfindung ist daher die Verwendung einer ÖI-in-Wasser-Emulsion mit lamellaren, flüssig-kristallinen Phasen, die eine kosmetische Öl- oder Fettkomponente, einen hydrophilen Emulgator und einen lipophilen Coemulgator enthält, der ein Lipid der allgemeinen Formel R¹ - O - R² ist, in der R¹ eine primäre, lineare Alkyl-, Alkenyl- oder Acylgruppe mit 20 bis 30 C-Atomen und R² Wasserstoff eine Gruppe der Formel - (CnH₂ₙO)ₓ - H, worin x = 1 oder 2 und n = 2 - 4 ist, oder eine Polyhydroxyalkylgruppe mit 4 - 6 C-Atomen und 2 - 5 Hydroxylgruppen ist, zur Wiederherstellung der Lamellarität und des Ordnungsgrades der epidermalen Lipidstrukturen der geschädigten Haut.

Diese Wirkung der erfindungsgemäßen, lamellaren Öl-in-Wasser-Emulsionen läßt sich experimentell durch infrarotspektroskopische Untersuchung der Konformationsordnung der - (CH₂)ₓ - Ketten der Lipide des Statum Corneum zeigen. Die Lage der Streckschwingungen νₛ (CH₂) und νₐₛ (CH₂) (ca. 2850 cm⁻¹ bzw. 2915 cm⁻¹) ist von dem Anteil der energetisch höher liegenden _{"}gauche"-Konformeren einer Lipidkette gegenüber den energetisch niedriger liegenden _{"}all-trans"-Konformeren abhängig. Steigende Unordnung der Lipidmembran führt zu einer Verschiebung dieser Banden zu höheren Frequenzen (bis zu einigen cm⁻¹) aufgrund der Zunahme des Anteils der energiereicheren Schwingungen der _{"}gauche"-Konformeren (vgl. RO. Potts, M.L. Francoeur: Infrared Spectroscopy of Stratum Corneum Lipids in: Pharmaceutical Skin Penetration Enhancement, ed. by Kenneth A. Walters, Jonathan Hadgraft, 1993, Seiten 269 - 291 ).

An einer durch Waschen mit Laurylsulfat-Lösung geschädigten Hautpartie konnte gezeigt werden, daß durch Behandlung mit einer erfindungsgemäßen Lamellarcreme in wenigen Tagen eine Zunahme der Konformationsordnung (_{"}Lamellarität") der epidermalen Lipide erreicht werden kann.

Die folgenden Beispiele sollen den Gegenstand der Erfindung näher erläutern:

### Beispiele

### Cremes mit Lamellarstruktur

### 1. Allgemeines Herstellverfahren

Die Öl- und Fettkomponenten. Emulgatoren; Coemulgatoren sowie die lipophilen Hilfsstoffe wurden gemischt und auf 95°C erwärmt.

Die wasserlöslichen Hilfsstoffe (Konservierungsmittel, Xanthan-Gum) wurden in Wasser gelöst. Die auf 90°C erwärmte wäßrige Phase wurde unter Rühren in die auf 90°C erwärmte Fettphase einemulgiert. Die dabei gebildete Emulsion wurde homogenisiert und dabei auf 40°C gekühlt. Nach Zugabe des Parfumöls wurde unter Rühren auf 20°C gekühlt.

Es wurden die folgenden Handelsprodukte verwendet:

| | | |
|---|---|---|
| (1) | Baysilonöl M 350 | Polydimethylsiloxan, 350 cst (25°C) |
| (2) | Lanette® 12 | Behenylalkohol technisch (C₂₂ : 70 - 80 %, C₂₀ : 10 - 20 %, C₁₈ : 5 - 15 %) |
| (3) | Controx®KS | Tocopherol/Talgefettsäure-glycerid-citrat-Gemisch |
| (4) | Citricidal® | Grapefruit-Samenextrakt |
| (5) | Dow Coming 344 Fluid | Octamethyl-cyclotetrasiloxan |
| (6) | Abil®-Wax 9809 | Polysiloxan-polyalkylen-Copolymer |
| (7) | Euxyl®K 400 | 1.2-Dibrom-2.4-dicyanobutan |
| (8) | Arlacel® 1689 | Polyglycerin-/sorbitan-Fettsäureester |
| (9) | Biophilic® S | Lecithin-Fettsäure-Fettalkohol-Gemisch |
| (10) | Arlacel® 989 | hydr. Rizinusöloxethylat (7 EO) |
| (11) | Gilugel min | Hydroxystearinsäure-Al/Mg-Salz/Paraffinöl |
| (12) | Glucolys® (Seporga) | Gemisch aus Glucose, Sorbit und Citronensäure |

### 2. Rezepturbeispiele

**Tabelle I**

| | **1** | **2** | **3** | **4** | **5** | **6** | **7** |
|---|---|---|---|---|---|---|---|
| **Ölphase :** | | | | | | | |
| Paraffinöl | - | - | 5,0 | 10,0 | 20,0 | - | - |
| Heptamethylnonan | - | - | - | - | - | 10,0 | 10,0 |
| Di-n-octylether | - | - | 5,0 | 10,0 | 20,0 | - | - |
| Nachtkerzenöl | 2,0 | 2,0 | - | - | - | - | - |
| Sonnenblumenöl | - | - | - | - | - | 10,0 | 10,0 |
| Mandelöl | - | 2,0 | - | - | - | - | - |
| Isopropyl-isostearat | 3,0 | 3,0 | - | - | - | - | - |
| Cetearyl-isononanoat | 2,0 | - | - | - | - | - | - |
| Baysilonöl M350 | 0,5 | 0,5 | - | - | - | - | - |
| Tocopherolacetat | 0,5 | 0,5 | - | - | - | - | - |
| Lanette 22 | 6,0 | 6,0 | 6,0 | 6,0 | 6,0 | 6,0 | 6,0 |
| pHB-Propylester | - | - | - | - | - | 0,1 | 0,1 |
| Controx KS | 0,05 | 0,05 | 0,05 | 0,05 | 0,05 | 0,05 | 0,05 |

| **Wäßrige Phase:** | | | | | | | |
|---|---|---|---|---|---|---|---|
| PEG 25 Sojasterol | 0,5 | 0,5 | - | - | - | - | - |
| Na-Cetyl-/Stearylsulfat | 0,24 | 0,24 | - | - | - | - | 0,1 |
| K-Cetylhydrogenphosphat | - | - | 0,1 | 0,1 | 0,1 | 0,1 | - |
| Xanthan-Gum | 0,05 | 0,05 | 0,3 | 0,3 | 0,3 | 0,1 | 0,1 |
| Dipropylenglycol | 5,0 | 5,0 | - | - | - | - | - |
| Glycin | 1,0 | 1,0 | - | - | - | - | - |
| Citricidal | - | - | - | - | - | 1,0 | 1,0 |
| Parfümöl | - | - | 0,2 | 0,2 | - | - | - |
| Wasser | ad 100 | ad 100 | ad 100 | ad 100 | ad 100 | ad 100 | ad 100 |

**Tabelle II**

| | **8** | **9** | **10** | **11** | **12** | **13** | **14** |
|---|---|---|---|---|---|---|---|
| **Ölphase:** | | | | | | | |
| Avocadoöl | 10,0 | 10,0 | 10,0 | - | - | - | - |
| Heptamethylnonan | 10,0 | 10,0 | 10,0 | - | - | - | 10,0 |
| Dow Corning 344 Fluid | - | - | - | 2,0 | - | - | - |
| Baysilonöl M 350 | - | - | - | - | 2,0 | - | - |
| Abil-Wax 9801 | - | - | - | - | - | 2,0 | - |
| Mikrowachs | - | 2,0 | - | - | - | - | - |
| Bienenwachs | 2,0 | - | - | - | - | - | - |
| Camaubawachs | - | - | 1,0 | - | - | - | - |
| Cetyl-/Stearylalkohol | - | - | - | - | - | - | 2,0 |
| Lanette 22 | 6,0 | 6,0 | 6,0 | 6,0 | 6,0 | 6,0 | 4,0 |
| pHB-Propylester | 0,1 | 0,1 | 0,1 | 0,1 | - | - | - |
| Controx KS | 0,05 | 0,05 | 0,05 | 0,05 | - | - | - |

| **Wäßrige Phase:** | | | | | | | |
|---|---|---|---|---|---|---|---|
| Na-Cetyl-/Stearylsulfat | 0,1 | 0,1 | 0,1 | - | - | - | 0,1 |
| K-Cetylhydrogenphosphat | - | - | - | 0,1 | 0,1 | 0.1 | - |
| Xanthan-Gum | 0,1 | 0,1 | 0,1 | 0,3 | 0,3 | 0,3 | 0,3 |
| Citricidal | 1,0 | 1.0 | 1,0 | - | - | - | - |
| Euxyl K 400 | - | - | - | - | - | 0,1 | - |
| 1.6-Hexandiol | - | - | - | 5,0 | - | - | - |
| Parfümöl | - | - | - | 0,2 | - | - | - |
| Wasser | 70,65 | 70,65 | 70,65 | 83,35 | 91,5 | 91,5 | 83,6 |

**Tabelle III**

| | **15** | **16** | **17** | **18** | **19** |
|---|---|---|---|---|---|
| **Ölphase:** | | | | | |
| Paraffinöl | - | - | 10,0 | - | 10.0 |
| Mandelöl | - | - | - | 10,0 | - |
| Heptamethylnonan | 10,0 | 6,7 | 10,0 | - | 10,0 |
| Decaglycerin-decaoleat | - | 0,0 | - | - | - |
| Baysilonöl M 350 | - | - | 0,5 | - | - |
| Tocopherolacetat | - | 2,0 | - | - | - |
| Sorbitan-monostearat | 2,0 | - | - | - | - |
| Arlacel 1689 | - | 1,0 | - | - | - |
| Biophilic S | - | - | 3,0 | - | - |
| Lanette 22 | 4,0 | 6,0 | 3,0 | 6,0 | 6,0 |
| pHB-Propylester | - | - | 0,1 | - | - |
| Controx KS | - | - | - | 0,05 | - |

| **Wäßrige Phase:** | | | | | |
|---|---|---|---|---|---|
| Na-Cetyl-/Stearylsulfat | 0,1 | - | - | - | - |
| K-Cetylhydrogenphosphat | - | 0,15 | 0,1 | 0,1 | 0,1 |
| Xanthan-Gum | 0,3 | 0,1 | 0,3 | 0,1 | - |
| Dipropylenglycol | - | - | 3,0 | - | - |
| Glucose | - | 0,2 | - | - | - |
| Euxyl K 400 | - | - | 0,1 | 0,1 | 0,1 |
| 1.6-Hexandiol | - | 10,0 | - | - | |
| Phenoxyethanol | - | - | 1,0 | - | - |
| Parfüm | - | 0,2 | 0,2 | 0,2 | - |
| Mg SO₄ | - | 0,2 | - | - | - |
| Wasser | 83,6 | | 68,7 | 83,55 | 73,8 |

**Tabelle IV**

| | **20** | **V** |
|---|---|---|
| **Ölphase:** | | |
| Paraffinöl | - | 10,0 |
| Isopropyl-isostearat | 7,5 | - |
| Isopropyl-palmitat | - | 5,0 |
| Mandelöl | 5,0 | 2,0 |
| Nachtkerzenöl | 2,0 | 2,0 |
| Baysilonöl M 350 | 0,5 | |
| Bienenwachs | - | 3,0 |
| Lanette 22 | 6,0 | - |
| Methylglucose-dioleat | - | 3,0 |
| Arlacel 989 | - | 0,1 |
| Sojasterin | - | 0,5 |
| Gilugel min | - | 3,0 |
| Tocopherolacetat | - | 2,0 |
| Controx KS | 0,05 | - |
| PHB-Propylester | 0,1 | 0,1 . |

| **Wäßrige Phase:** | | |
|---|---|---|
| Na-Cetyl-Stearylsulfat | 0,18 | - |
| Xanthan-Gum | 0,05 | - |
| Mandelprotein | 1,5 | 1,5 |
| Bisabolol | 0,1 | 0,1 |
| Glycolys | 1,0 | 1,0 . |
| MgSO₄, | - | 1,0 |
| Glyerin | - | 3,0 |
| Propylenglycol | 3,0 | - |
| PHB-Methylester | 0,3 | 0,3 |
| Euxyl K 400 | 0,2 | 0,2 |
| Parfümöl | 0,37 | 0,37 |
| Wasser | 72,11 | 61,83 |

### 3. IR-spektroskopische Untersuchung der Effekte auf das Stratum Corneum

| **Testablauf:** | |
|---|---|
| Prüfcremes | Creme Nr. 20 und W/O-Vergleichscreme V |
| Probanden | Zwei Gruppen zu je 10 Probanden |
| Anwendung | Morgens und abends jeweils am Unterarm |
| Messung | FT-IR-ATR, Zn Se-Kristall, linker Arm unbehandelt (Referenz), rechter Arm behandelt. Die Meßwerte wurden durch Differenzbildung zum Nullwert ermittelt. |
| Meßzeitpunkte | 1.) Nullwert vor der 1. Anwendung 2.) 12 Std. nach der 28. Anwendung (2-Wochen-Kontrolle) |

Zunächst wurde bei allen Versuchspersonen für jeden Arm ein Nullwert bestimmt. Dann wurde jeweils auf die Innenseite des rechten Unterarms die zu prüfende Creme aufgetragen. Diese Cremebehandlung wurde an 14 Tagen jeweils morgens und abends durchgeführt. Es wurden jeweils Differenzen zwischen den Meßwerten des linken (unbehandelten) und des rechten (behandelten) Arms ermittelt. Aus diesen Differenzen wurden für jede der beiden Probandengruppen die Mittelwerte errechnet.

Dabei wurde festgestellt, daß die Lage der assymmetrischen CH₂-Streckschwinungen νₐₛ CH₂ bei der mit der Creme Nr. 20 behandelten Gruppe um 0,2 cm⁻¹ niedriger lag als der Wert für den nichtbehandelten linken Unterarm.

Bei der mit einer üblichen W/O-Creme (Rezeptur V) behandelten Gruppe war die Lage der assymmetrischen CH₂-Streckschwingung νₐₛ CH₂ hingegen um 0,1 cm⁻¹ höher als der Wert für den unbehandelten linken Arm.

Die Werte waren bei 95 %iger Wahrscheinlichkeit statistisch signifikant.

Die Messungen lassen den Schluß zu, daß die mit der erfindungsgemäßen Creme Nr. 20 behandelten Haut einen höheren Ordnungsgrad, d.h. einen geringeren Anteil energetisch höher liegender _{"}gauche-Konforinere" aufweist, als die mit der üblichen W/O-Creme behandelte Haut.

## Patentansprüche

1. Öl-in-Wasser-Emulsionen mit lamellaren, flüssig-kristallinen Phasen, enthaltend eine kosmetische Öl- oder Fettkomponente, einen hydrophilen Emulgator und einen lipophilen Coemulgator, **dadurch gekennzeichnet, daß** als lipophiler Coemulgator ein Lipid der allgemeinen Formel R¹ - O - R² enthalten ist, in der R¹ eine primäre lineare Alkyl-, oder Alkenylgruppe mit 20 bis 30 C-Atomen und R² Wasserstoff ist.

2. Öl-in-Wasser-Emulsion nach Anspruch 1 **dadurch gekennzeichnet, daß** als lipophiler Coemulgator Behenylalkohol in einer Menge von 20 - 80 Gew.-% der gesamten Ölphase enthalten ist.

3. Öl-in-Wasser-Emulsion nach einem der Ansprüche 1 bis 2, **dadurch gekennzeichnet, daß** als hydrophiler Emulgator ein ionischer oder ein nichtionischer Emulgator mit einem HLB-Wert von 8 - 18 enthalten ist, wobei der HLB-Wert sich ergibt aus HLB = 0,2 x (100 - L), wobei L der prozentuale Gewichtsanteil der lipophilen Alkyl-, Alkenyl- oder Acylgruppen im Molekül ist.

4. Verwendung eines Lipids der allgemeinen Formel R¹-O-R², in der R¹ eine lineare Alkyl- oder Alkenylgruppe mit 20 - 30 C-Atomen und R² Wasserstoff ist, als lipophilen Emulgator zur Herstellung einer Öl-in Wasser-Emulsion mit lamellaren, flüssig kristallinen Phasen. die eine kosmetische Öl- oder Fettkomponente, einen hydrophilen Emulgator und einen lipophilen Emulgator enthält, zur Wiederherstellung der Lamellarität und des Ordnungsgrades der epidermalen Lipidstrukturen der geschädigten Haut.

5. Verfahren zur Herstellung von Öl-in-Wasser-Emulsionen., kosmetischer Öl- oder Fettkomponenten, die lamellare, flüssig-kristalline Phasen aufweisen, unter Verwendung hydrophiler Emulgatoren und lipophiler Coemulgatoren, **dadurch gekennzeichnet, daß** man die wäßrige Phase, die hydrophile Emulgatoren enthalten kann, mit der Fettphase, die als lipophilen Coemulgator wenigstens ein Lipid der allgemeinen Formel R¹ - O - R² enthält, in der R¹ eine lineare Alkyl-oder Alkenylgruppe mit 20 bis 30 C-Atomen und R² Wasserstoff ist, intensiv vermischt.

## Claims

1. Oil-in-water emulsion with lamellar liquid crystalline phases containing a cosmetic oil or fatty component, a hydrophilic emulsifier and a lipophilic co-emulsifier, **characterized in that** the lipophilic co-emulsifier. used is a lipid corresponding to the general formula R¹-O-R², where R¹ is a primary linear alkyl or alkenyl group containing 20 to 30 carbon atoms and R² is hydrogen.

2. An oil-in-water emulsion as claimed in claim 1, **characterized in that** behenyl. alcohol is present as the lipophilic co-emulsifier in a quantity of 20 to 80% by weight, based on the oil phase as a whole.

3. An oil-in-water emulsion as claimed in claims 1 and 2, **characterized in that** the hydrophilic emulsifier is an ionic or nonionic emulsifier with an HLB value of 8 to 18, the HLB value being calculated in accordance with the equation HLB = 0.2 x(100 - L), where L is the percentage by weight of lipophilic alkyl, alkenyl or acyl groups in the molecule.

4. The use of a lipid corresponding to the general formula R¹-O-R², where R¹ is a primary linear alkyl or alkenyl group containing 20 to 30 carbon atoms and R² is hydrogen, as a lipophilic emulsifier for the production of an oil-in-water emulsion with lamellar liquid crystalline phases containing a cosmetic oil or fatty component, a hydrophilic emulsifier and a lipophilic co-emulsifier for restoring the lamellarity and degree of order of the epidermal lipid structures of damaged skin.

5. A process for the production of oil-in-water emulsions of cosmetic oil or fatty components containing lamellar liquid crystalline phases using hydrophilic emulsifiers and lipophilic co-emulsifiers, **characterized in that** the aqueous phase which may contain hydrophilic emulsifiers is intensively mixed with the fatty phase which contains as lipophilic co-emulsifier at least one lipid corresponding to the general formula R¹-O-R², where R¹ is a linear alkyl or alkenyl or acyl group containing 20 to 30 carbon atoms and R² is hydrogen.

## Revendications

1. Émulsions du type huile-dans-l'eau comprenant. des phases liquides-cristallines lamellaires, contenant un composant cosmétique huileux ou gras, un émulsifiant hydrophile et un coémulsifiant lipophile, **caractérisées en ce qu'**elles contiennent, à titre de coémulsifiant lipophile, un lipide répondant à la formule générale R¹ - O - R² dans laquelle R¹ représente un groupe alkyle ou un groupe alcényle linéaire primaire contenant de 20 à 30 atomes de carbone et R² représente un atome d'hydrogène.

2. Émulsion du type huile-dans-l'eau selon la revendication 1 **caractérisée en ce qu'**elle contient, à titre de coémulsifiant lipophile, de l'alcool béhénylique en une quantité de 20 à 80 % en poids de la phase huileuse totale.

3. Émulsion du type huile-dans-l'eau selon l'une quelconque des revendications 1 ou 2, **caractérisée en ce qu'**elle contient, à titre d'émulsifiant hydrophile, un émulsifiant ionique ou un émulsifiant non ionique possédant une valeur HLB de 8 - 18, la valeur HLB étant obtenue à partir de HLB = 0,2 x (100 - L) où L représente la fraction pondérale en % des groupes alkyle, alcényle ou acyle lipophiles dans la molécule.

4. Utilisation d'un lipide répondant à la formule générale R¹ - O - R² dans laquelle R¹ représente un groupe alkyle ou un groupe alcényle linéaire contenant de 20 à 30 atomes de carbone et R² représente un atome d'hydrogène, à titre d'émulsifiant lipophile pour la préparation d'une émulsion du type huile-dans-l'eau comprenant des phases liquides-cristallines lamellaires, qui contient un composant cosmétique huileux ou gras, un émulsifiant hydrophile et un émulsifiant lipophile, pour le rétablissement de la structure lamellaire et du degré d'ordre des structures lipidiques épidermiques de la peau endommagée.

5. Procédé pour la préparation de composants cosmétiques huileux ou gras d'émulsions du type huile-dans-l'eau, qui présentent des phases liquides-cristallines lamellaires, en utilisant des émulsifiants hydrophiles et des coémulsifiants lipophiles, **caractérisé en ce qu'**on soumet à un mélange intime la phase aqueuse, qui peut contenir des émulsifiants hydrophiles, avec la phase grasse, qui contient, à titre de coémulsifiant lipophile, au moins un lipide répondant à la formule générale R¹ - O - R² dans laquelle R¹ représente un groupe alkyle ou un groupe alcényle linéaire contenant de 20 à 30 atomes de carbone et R² représente un atome d'hydrogène.
